# EUROPEAN PATENT SPECIFICATION

(11) **EP 1 545 361 B1**
(45) Date of publication and mention of the grant of the patent: **28.03.2007**
(21) Application number: 03808089.1
(22) Date of filing: 11.09.2003
(51) Int. Cl.: A61B 18/14

(54) **ELECTROSURGICAL INSTRUMENT FOR SEALING VESSELS**
ELEKTROCHIRURGISCHES INSTRUMENT ZUM VERSCHLUSS VON GEFÄSSEN
INSTRUMENT ELECTROCHIRURGICAL POUR SOUDER DES VAISSEAUX

(30) Priority: 04.10.2002 US 416382 P
(43) Date of publication of application: 29.06.2005
(73) Proprietor: Sherwood Services AG, 8200 Schaffhausen (CH)
(72) Inventor: JOHNSON, Kristin, D., Louisville, CO 80027 (US); COUTURE, Gary, M., Longmont, CO 80501 (US); DYCUS, Sean, T., Denver, CO 80205 (US)
(74) Representative: Wiedemann, Peter
(86) International application number: PCT/US2003/028534
(87) International publication number: WO 2004/032776

(56) References cited:
- EP-A- 0 589 555
- EP-A- 1 177 771
- US-A- 6 106 542
- US-A1- 2002 091 385
- US-B1- 6 458 130

## Description

### BACKGROUND OF THE INVENTION

### Field of the Invention

This invention relates to an electrosurgical instrument for permanently closing vessels in a human or animal, and more particularly to a bipolar electrosurgical instrument that seals vessels and vascular tissue by applying a combination of pressure and electrosurgical current and maintaining an appropriate gap distance between vessel sealing surfaces during the sealing process.

A hemostat or some similar pliers-like tool is commonly used in surgical procedures to grasp, dissect and clamp tissue. A hemostat uses mechanical action between its jaws to constrict vessels without cutting them. It is also typical for hemostats to have an interlocking ratchet between the handles so that the device can be clamped and locked in place.

Many hemostats are used in a typical open-surgical procedure. Once vascular tissue has been clamped with a hemostat, it is common for a surgeon to tie a suture around the tissue to close it off permanently prior to removing the hemostat. Several hemostats may be left in the surgical field until the surgeon has the opportunity to tie a suture around each section of clamped tissue.

Small blood vessels have been closed using electrosurgical instruments without the need for sutures. For example, neurosurgeons have used bipolar instruments to coagulate vessels in the brain that are smaller than two millimeters in diameter. These bipolar instruments are typically tweezers-like devices with two arms that can be deflected toward each other to grasp tissue. However, it has been found that these instruments are not capable of sealing blood vessels with diameters larger than about two millimeters. There has been a long-felt need for an easy way to seal larger vessels and vascular tissue bundles without the need for sutures.

It is thought that the process of coagulating small vessels is fundamentally different than vessel sealing. Coagulation is defined as a process of desiccating tissue wherein the tissue cells are ruptured and dried. Vessel sealing is defined as the process of liquefying the collagen in the tissue so that it crosslinks and reforms into a fused mass. Thus, coagulation of small vessels is sufficient to permanently close them. Larger vessels need to be sealed to assure permanent closure.

A number of bipolar electrosurgical forceps and clamps are known in the field. An example of a bipolar electrosurgical power curve for vessel sealing is disclosed in U.S. Pat. No. 5,827,271, and is hereby incorporated by reference and made a part of this disclosure.

U.S. Pat. No. 5,776,130 discloses another surgical tool for sealing vessels, and is hereby incorporated by reference and made a part of this disclosure. U.S. Pat. No. 371,664 discloses a pair of electric forceps with positive and negative electric poles located on the jaws. U.S. Pat. No. 728,883 discloses an electrothermic instrument in which electricity is used to heat one of the jaws of the instrument. U.S. Pat. No. 1,586,645 discloses a bipolar instrument for coagulating tissue. U.S. Pat. No. 2,002,594 discloses a bipolar laparoscopic instrument for treating tissue, whereby coagulation and cutting of tissue can be performed with the same instrument. U.S. Pat. No. 2,176,479 discloses an instrument for finding and removing metal particles. The jaws of the instrument are designed to complete an electrical circuit when conductive material is placed therebetween. An insulated pivot and an insulated ratchet are used to prevent a short circuit. U.S. Pat. No. 3,651,811 discloses a bipolar electrosurgical instrument for cutting and coagulating tissue. U.S. Pat. No. 4,005,714 discloses bipolar coagulation forceps with jaws that open and close by way of an actuating sleeve. U.S. Pat. Nos. 4,370,980 and 5,116,332 disclose an electrocautery hemostats wherein the hemostatic clamping function and the electrocautery function may be accomplished with a single instrument. Monopolar electrosurgical designs are shown and described.

U.S. Pat. No. 4,552,143 discloses a family of removable switch electrocautery instruments, including an electrocautery hemostat. Monopolar electrosurgical designs are shown and described. U.S. Pat. No. 5,026,370 discloses an electrocautery forceps instrument having an enclosed electrical switching mechanism. Monopolar electrosurgical designs are shown and described. U.S. Pat. No. 5,443,463 discloses coagulating forceps having a plurality of electrodes. U.S. Pat. No. 5,484,436 discloses bipolar electrosurgical instruments for simultaneously cutting and coagulating tissue. Russian Patent 401,367 discloses a bipolar instrument with a linkage that brings the working jaws together in a parallel manner.

An electrosurgical instrument according to the preamble of claim 1 is disclosed in EP-A-0589555. In that instrument, the outer clamping member is formed by a pivoting handle which by its pivotal movement moves an inner tube in a longitudinally reciprocating fashion. A pair of conductive leads extends through the entire length of the inner tube. The leads terminate at the distal end of the inner tube in a pair of forceps jaws provided with seal surfaces. By longitudinally moving the inner tube in a distal direction the seal surfaces are moved towards one another for grasping tissue therebetween. Accordingly, the mechanism for moving the seal surfaces towards and from one another is quite different from the mechanism of the invention.

From EP-A-1177771 a medical instrument for use in combination with endoscopes is known. The instrument includes two handles, which are connected to the halves of a tong, and one handle and one half of the tong are connected by a rod, resulting in a static and a moving tong surface.

As far as is known, prior disclosures have not provided a design for an electrosurgical instrument capable of establishing a separation distance between the seal surfaces by at least one stop in the instrument and of conveniently applying a constant pressure, via a push-rod mechanism controlled by a ratchet that is sufficient to seal vessels and vascular tissue during surgical procedures, and where the separation distance is controlled at a proximal end of the instrument rather than at a distal end.

### SUMMARY

It is the general objective of this invention to provide an electrosurgical instrument that can fuse tissue without the need for a suture or surgical clips. The instrument conducts electrosurgical current between two seal surfaces located on opposable jaws. The electrosurgical current passes through tissue clamped between the jaws and remolds the collagen to fuse the tissue and form a permanent seal.

One advantage of the invention is that blood vessels can be quickly fused and permanently sealed against passage of blood or other fluids. The instrument thereby reduces operating-room time, provides improved access to target tissues, and increases the efficiency of the surgical procedure.

Another advantage is that no sutures or staples are required to permanently seal blood vessels, and no foreign material is left in the body of the patient.

Yet another advantage is that vessels can be sealed as the instrument is applied, and then the instrument can be removed from the surgical field. This keeps the surgical field clear of extraneous tools that may hinder the surgeon's access to the surgical site.

Yet another advantage is that the proper amount of pressure can be applied by the instrument to the vessel or vessels, thereby increasing the likehood of a successful surgical outcome.

The invention is defined in claim 1 below. The dependent claims are directed to optional and preferred features.

The electrosurgical instrument of the present invention is preferably bipolar and designed for minimally invasive surgical procedures. The instrument may include a stationary inner member and an outer clamping member having movable parts connected to the inner member by an open lockbox, interlocking ratchet teeth, and electrical terminals with conductive pathways leading to opposable jaw members. The inner member and outer clamping member each have a ring handle near a proximal end of the instrument and the pivoting jaw members have opposable seal surfaces near a distal end of the instrument. The proximal end is held and controlled by the surgeon, while the distal end is used to manipulate tissue.

The open lockbox joins the inner member and the outer clamping member to allow for providing push-rod pivot motion via a push-rod mechanism of the outer clamping member and for arcuate motion of the jaw member of the outer clamping member. The open lockbox is generally designed to provide lateral support so that both seal surfaces align in approximately the same plane. The seal surfaces are preferably aligned opposite each other when the sealing jaws are closed together, i.e., when a laterally-moving push-rod of the push-rod mechanism is translated distally to push one of the sealing jaws towards the opposing sealing jaw to seal or fuse vessels, tissue and the like.

As the push-rod is translated distally, the push-rod eventually abuts a stop or protrusion on a proximal end of the inner member preventing the push-rod from being further advanced. When this occurs the movable sealing surface and the stationary sealing surface are in close proximity to each other without contacting each other, thereby preventing a short circuit. Preferably, the stop maintains a minimum separation distance of at least about .03 millimeters to about 0.15 millimeters between the seal surfaces.

To provide lateral support, the open lockbox includes a pivot pin and at least one flange or connecting member extending over the inner member and attached to the outer clamping member. The connecting member further connects the laterally-moving push-rod to a pivoting support member of the push-rod mechanism.

The instrument is tuned to provide a proper closure force by adjusting the dimensions of a shank portion on the inner member and the outer clamping member. The shank portion is defined as the portion of the inner member and the outer clamping member bounded by their respective ratchet stub and the open lockbox. During use, the surgeon squeezes the ring handles to compress tissue between the seal surfaces. The shank portion of the inner member and the outer clamping member flexes in the manner of a cantilever spring, and can be locked in a deflected position with the ratchet to hold a constant force. It is one of the objects of the invention to provide a range of ratchet stops that correspond to a range of appropriate closure forces on the seal surfaces of the instrument.

Ratchet teeth are located on the inner member and the outer clamping member near their respective ring handles. The ratchet teeth are generally designed to interlock against the spring force from the shanks. The spring force is thus transmitted through the pivot to hold the seal surfaces against each other. A range of closure forces is required in an instrument, depending on the type and thickness of the tissue to be sealed. It is thus desirable to have several ratchet stops, each providing a progressively larger force to the seal surfaces.

An electrical connector is located on one or both the ring handle, shank, ratchet or other part of the instrument. The electrical connector may be a metal post that is integrally formed with the ring handle. Bipolar electrical cables from an electrosurgical generator are connected to the instrument at the electrical connectors. An electrically conductive path on each of the inner member and the outer clamping member conducts the electrosurgical current to the seal surfaces. The electrically conductive paths may be along the stainless steel inner member and the outer clamping member. An electrically, insulative coating is preferably bonded to the surfaces of at least one of the inner member and outer clamping member to protect the surgeon and patient against inadvertent electrical burns. It is also contemplated that the instrument can be monopolar where either the inner member or the outer clamping member is provided with the electrically conductive path for energizing only one of the seal surfaces.

The following terms are herein defined as follows. The applied force of the instrument is the total force being applied to the tissue between the jaws. The jaws are the members at the distal end of the instrument. The seal surface is the feature on the inner surface of the jaws that comes in direct contact with the tissue. The shanks are the portions of the inner member and the outer clamping member between the lockbox and the ratchet. The ring handles are the elements on the inner member and the outer clamping member, near the proximal end of the instrument that are grasped by the surgeon. The lockbox is the structure that allows the outer clamping member to pivot with respect to the inner member, including the pivot pin and other cooperating surfaces. The inner member is the member that remains stationary or is generally captured in the interior of the lockbox. The outer clamping member includes movable parts and is on the outside of the lockbox. Jaw pressure is calculated by dividing the applied force over the complete area of the seal surface. Tissue pressure is calculated by dividing the applied force over the area of tissue placed between the jaws.

It has been found through experimentation that an instrument for vessel fusion (also referred herein as vessel sealing) should compress the tissue with a proper amount of pressure between the sealing jaws. The pressure is preferably sufficient to close any blood-carrying lumen. The pressure is preferably low enough so that the tissue is not split apart within the sealing jaws.

The jaws of the instrument should not short-circuit during the procedure. The-tissue will typically decrease in thickness when electrosurgical current is applied, thereby allowing the seal surfaces to move closer together. This decrease in thickness shouldn ot result in the jaws making direct contact with each other. Otherwise, a short circuit could give the electrosurgical current a preferential path around the tissue and may result in a poor seal. To prevent the jaws from making direct contact with each other, at least one stop is provided at a proximal end of the instrument to establish a gap between the sealing surfaces of the jaws.

More particularly, the present disclosure includes an electrosurgical instrument for sealing tissue having an inner member and an outer clamping member at least one of which includes a ring handle near a proximal end thereof. Each of the inner member and the outer clamping member includes a seal surface proximate a distal end thereof. At least one of the inner member and the outer clamping member are movable from a first position wherein the seal surfaces are spaced relative to one another to a second position wherein the seal surfaces are disposed in opposing relation relative to one another for grasping tissue therebetween.

A rod is operably connected at a proximal end to the outer clamping member and is operably connected at a distal end to the seal surface of the outer clamping member. The rod is translatable to pivot the seal surface of the outer champing member relative to the seal surface of the inner member. Advantageously, the rod is operatively connected to a pivoting arm which, in turn, is operatively connected to the outer clamping member such that movement of the outer clamping member pivots the pivoting arm to move the rod.

A stop is included and is located near the proximal end of the inner member for limiting distal movement of the push rod and to maintain a gap between the opposing seal surfaces when the seal surfaces are disposed in the second position. The seal surfaces of the the electrosurgical instrument are also connectable to a source of electrosurgical energy such that electrosurgical energy can be communicated through tissue held therebetween when the seal surfaces are disposed in the second position to form a tissue seal. Preferably, the stop maintains a separation distance between the sealing surfaces in the range of about 0.03 millimeters to about 0.15 millimeters. In one particularly advantageous embodiment, the stop member is selectively positionable to vary the separation distance between the sealing surfaces. In another embodiment, the stop member is selectively replaceable to vary the separation distance between the seating surfaces.

The inner member includes a shank portion and the outer clamping member includes a shank portion which cooperate to provide a mechanically advantageous spring load during tissue clamping. The inner member and the outer clamping member each include interfacing ratchets which cooperate to hold a closure force at discrete positions, preferably within the a range of about 3 Kg/cm² to about 16 Kg/cm².

In another particularly advantageous embodiment, the instrument includes a cutting mechanism which is selectively actuateable to sever tissue after formation of the tissue seal.

In accordance with another embodiment according to the present disclosure, the electrosurgical instrument includes the inner member and the outer clamping member which are pivotable within an open lockbox to move from a first position wherein the seal surfaces are spaced relative to one another to a second position wherein the seal surfaces are disposed in opposing relation relative to one another for grasping tissue therebetween. Preferably, the open lockbox includes a bottom surface disposed on one of the inner member and the outer clamping members which matingly receives a corresponding surface disposed on the other of the inner member and the outer clamping member. A pivot is preferably disposed through the bottom surface of the open lockbox for joining the inner member and the outer clamping member for pivotable rotation thereabout.

Further features of the electrosurgical instrument of the invention will become more readily apparent to those skilled in the art from the following detailed description of the apparatus taken in conjunction with the drawings.

### BRIEF DESCRIPTION OF THE DRAWINGS

Various embodiments of the electrosurgical instrument of the invention will be described herein below with reference to the drawings wherein:
FIG. 1 is a perspective view of the electrosurgical instrument for vessel fusion in accordance with one preferred embodiment with jaws spaced-apart, shown partially exploded;
FIG. 2 is a side view of the electrosurgical instrument of FIG. 1;
FIG. 3 is an enlarged view of the area of detail encircled in FIG. 2 and labeled 3 showing a stop at the distal end of the electrosurgical instrument;
FIG. 4 is an enlarged view of the area of detail encircled in FIG. 2 and labeled 4 showing the proximal end of a push-rod mechanism;
FIG. 5 is a perspective view of the electrosurgical instrument for vessel fusion in accordance with another preferred embodiment having a cutting mechanism;
FIG. 6 is a side view of the electrosurgical instrument of FIG. 5 with a cut activator of the cutting mechanism being actuated to cut tissue;
FIG. 7 is a side view of the electrosurgical instrument for vessel fusion in accordance with another preferred embodiment utilizing plugs at the proximal end of the electrosurgical instrument; and
FIG. 8 is a side view of the electrosurgical instrument for vessel fusion in accordance with another preferred embodiment utilizing a screw-like mechanism at the proximal end of the electrosurgical instrument.

### DETAILED DESCRIPTION OF PREFERRED EMBODIMENTS

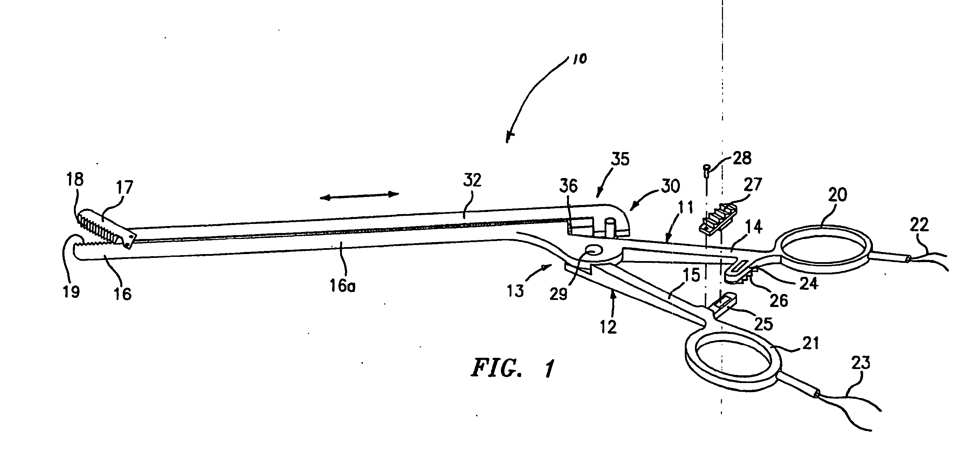

The present invention provides an electrosurgical instrument that can fuse tissue without the need for a suture or surgical clips. With reference to FIGS. 1-4, the instrument 10 has a stationary inner member 11 and an outer clamping member 12. The inner member 11 and outer clamping member 12 are connected through an open lockbox 13 which has a gap between flanges 33. The terms "inner" and "outer" are used to distinguish the inner member 11 and outer clamping member 12, and their component parts, according to their respective positions at the open lockbox 13. The inner member 11 is fitted generally within the inner surfaces of the open lockbox 13 and is captured by the flanges 33. The outer clamping member 12 generally forms the outside surfaces of the open lockbox 13.

The inner member 11 has an inner shank 14, an inner jaw 16, an elongated member 16a, and an inner ring handle 20. In the preferred embodiment, the inner shank 14, the inner jaw 16, the elongated member 16a, and the inner ring handle 20 are unitary. Similarly, the outer clamping member 12 has an outer shank 15, an outer jaw 17, an outer ring handle 21, and a push-rod mechanism 30 operatively connecting the outer jaw 17 to the outer shank 15. The ring handles, 20 and 21, are designed for a surgeon to hold and manipulate the instrument 10. The jaws, 16 and 17, are designed to grasp tissue between the opposing seal surfaces 18 and 19. The jaw 16 is stationary, while the jaw 17 is movable.

Each shank, 14 and 15, has a respective ratchet stub 24 or 25. Ratchet teeth, 26 and 27, are designed to interlock in a manner that holds the inner member 11 and outer clamping member 12 in position. The shanks 14 and 15 are deflected in the manner of a cantilever spring when the jaws are forced together by the surgeon. The deflection of the shanks 14 and 15 produces a spring restoring force that can be opposed by interlocking the ratchet teeth, 26 and 27.

The instrument 10 does not cause a short circuit when the ratchet teeth, 26 and 27, are interlocked. This is accomplished by a suitable selection and placement of electrically insulating materials. In the preferred embodiment, the ratchet teeth 26 and 27 are composed of a polymeric material which is press-fit into the ratchet stubs 24 and 25. A ratchet screw 28 is used in the preferred embodiment to secure the ratchet teeth 26 and 27 into the ratchet stubs 24 and 25 (see FIG. 1). During manufacture, the ratchet teeth 26 and 27 may be formed from a blank after the blank has been press fit into the ratchet stubs 24 and 25.

The open lockbox 13 has the function of providing a pivoting joint for the inner member 11 and the outer clamping member 12. In addition, the flanges 33 provide lateral support to help maintain alignment of the jaws 16 and 17. Closed lockbox designs are typically used in standard hemostat designs, wherein an inner member is completely captured through a slot in an outer member. The open lockbox 13 in present invention has a gap between the flanges 33 that is different from a closed lockbox design. The gap in the open lockbox 13 provides convenient access to install an electrically insulated pivot.

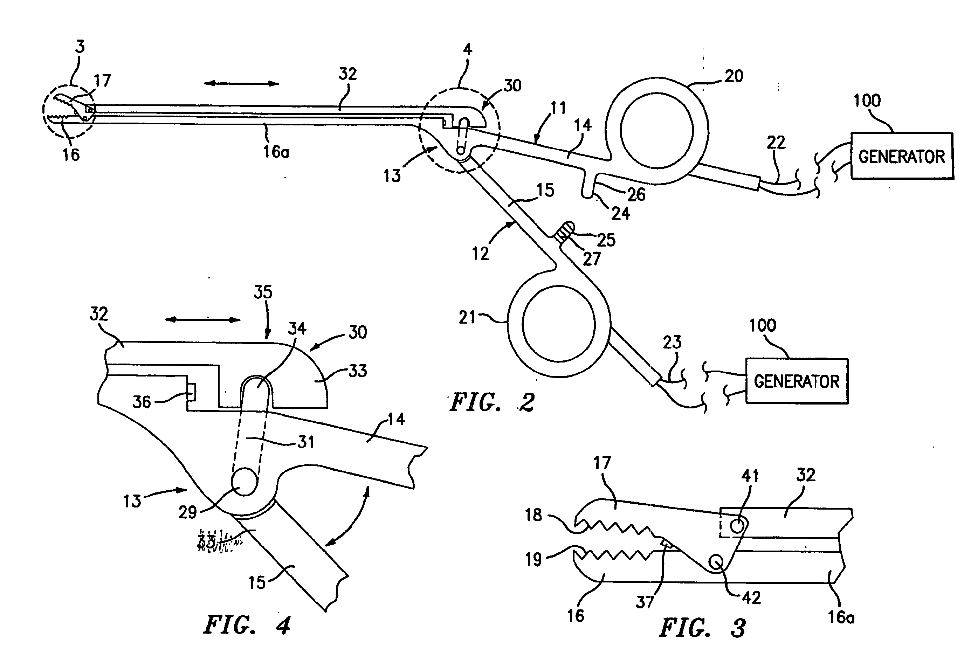

The electrically insulated pivot in the present invention includes standard pin 29 for connecting the inner member 11 and the outer clamping member 12 and keeping the inner member 11 stationary with respect to the outer clamping member 12. The pin 29 further connects the push-rod mechanism 30 of the outer clamping member 12 to a portion of the outer shank 15. The push-rod mechanism 30 includes a pivoting arm 31 held to the outer shank 15 by the pin 29 and to a push-rod 32 by a ball-and-socket arrangement 34 at a proximal end 35 of the push-rod. The pin 29 and the pivoting arm 31 are composed of or coated with electrically insulative material that prevents a short circuit between the inner member 11 and the outer clamping member 12. A proximal portion of the outer jaw 17 is connected to a distal portion of the push-rod 32 by a pin 41 and to the inner member 11 by a pin 42 (see-FIG. 3). The proximal portion of the outer-jaw 17 and-the pin 42 are composed of or coated with electrically insulative material.

During operation, as the push-rod 32 is translated distally, the outer jaw 17 pivots counter-clockwise with respect to pin 42 and is simultaneously pushed distally by the push-rod 32 via pin 41. The push-rod 32 eventually abuts a stop or protrusion 36 on the inner member 11 preventing the push-rod 32 from being further advanced. When this occurs the movable sealing surface 19 of outer jaw 17 and the stationary sealing surface 18 are in close proximity to each other without contacting each other, thereby preventing a short circuit. Preferably, the stop 36 maintains a minimum separation distance of at least about .03 millimeters to about 0.15 millimeters between the seal surfaces 18 and 19. In an alternate embodiment, rather than using the stop 36, the inner member 11 is dimensioned accordingly to enable the push-rod 32 to abut it to maintain a minimum separation distance of at least about .03 millimeters to about 0.15 millimeters between the seal surfaces 18 and 19.

The inner member 11 and the outer clamping member 12 are connected to a respective pole of a bipolar electrosurgical generator 100. Electrical connectors 22 and 23 are located on the ring handles 20 and 21 to provide a convenient point of connection. The inner member 11 and the parts of the outer clamping member 12 are formed of an electrically conductive material, such as stainless steel. The exposed surfaces of at least one of the inner member 11 and the outer clamping member 12, except for the connectors 22 and 23 and the seal surfaces 18 and 19, are preferably spray coated with an insulating material.

The characteristics of the bipolar electrosurgical current are determined by the design of the electrosurgical generator. In the preferred embodiment, the generator will have an output wherein the peak-to-peak voltage will not exceed 130 Volts. This is because higher voltages can cause sparking which results in localized burning of tissue which may result in a failure of the tissue weld. The preferred embodiment has the generator capable of producing high frequency output current of at least 2 Amps RMS. High electrical current is important because it heats the tissue sufficiently to melt the collagen. Lower electrical currents will often produce weak tissue welds with low bursting strength.

During operation, the instrument 10 is used to grasp tissue between the seal surfaces 18 and 19. The surgeon squeezes the ring handles 20 and 21 together, causing pressure to be applied to the tissue. The ratchet teeth 26 and 27 are interlocked at the appropriate ratchet setting, depending on the tissue type and tissue thickness. Bipolar electrosurgical current is applied through the instrument 10 and the tissue to cause the tissue to fuse into single mass. It is also contemplated that the instrument can be monopolar where either the inner member 11 or the outer clamping member 12 is provided with an electrically conductive path for energizing only one of the seal surfaces 18 and 19.

The jaws 16 and 17 have a structure and cross-section that resist bending under load. Thus, for purposes of engineering analysis, the shank portions 14 and 15 act as a cantilever supported beam once the seal surfaces 18 and 19 have been opposingly positioned. The length of this idealized cantilever beam extends from the push rod mechanism 30 to the location of the respective ratchet subs 24 or 25. It is possible to model each shank as a cantilever spring having a spring constant. Each-ratchet position is designed to transmit a particular closure force to the jaws 16 and 17 against the action of the restoring force of the cantilever spring.

The spring constant is generally a function of Young's Modulus of the shank material, the moment of inertia of the shank, and the length of the shank portion 14 and 15. When the jaws 16 and 17 of the instrument 10 are closed together, each shank 14 and 15 approximates a cantilever-supported beam. It is properly assumed that the deflection of each shank 14 and 15 remains within the linear range of its stress-strain curve. The behavior of such a beam is well known to materials engineers. A large spring constant will result in large closure forces between the seal surfaces 18 and 19. Similarly, a small spring constant will result in a small closure forces between the seal surfaces 18 and 19. The choice of a proper spring constant will depend on the length of the shank 14 or 15 and the distance between ratchet stops 26 and 27.

Experimental results studies suggest that the magnitude of pressure exerted on the tissue by the seal surfaces 18 and 19 is important in assuring a proper surgical outcome. Tissue pressures within a working range of about 3 kg/cm² to about 16 kg/cm² have been shown to be effective for sealing arteries and vascular bundles. It is desirable to tune the spring constant of the shank portions 14 and 15, in conjunction with the placement of the ratchet teeth 26 and 27, such that successive or discrete ratchet positions will yield pressures within the above working range. In one embodiment, the successive ratchet positions are two millimeters apart.

Pressure on the tissue can be described in several ways. Engineers will recognize that the amount of pressure exerted on the tissue depends on the surface area of the tissue that is in contact with the seal surfaces. In the one embodiment, the width of each seal surface 18 is in the range of 2 to 5 millimeters, and preferably 4 millimeters width, while the length of each seal surface 18 and 19 is preferably in the range of 5 to 20 millimeters. It has been found through experimentation that at least one interlocking ratchet position preferably holds the closure force between approximately 400 and 650 grams per millimeter of seal surface width. For example, if the width of the seal surface 18 and 19 is 4 millimeters, the closure force is preferably in the range of 1600 grams to 2600 grams. In one embodiment, the closure force is 525 grams per millimeter of width, yielding a closure force of 2100 grams for a 4 millimeter width seal surface 18 and 19.

In the preferred embodiment, a stop 37, made from insulative material, is located at the proximal end of the outer jaw 17 of the instrument 10 to assist the protrusion 36 in maintaining a minimum separation distance of at least about .03 millimeters to about 0.15 millimeters between the seal surfaces 18 and 19, as shown in FIG. 3. The stop 37 reduces the possibility of short circuits between the seal surfaces 18 and 19. As can be appreciated, stop 36 may act alone to maintain the appropriate gap distance between opposing seal surfaces 18 and 19 during sealing.

A method of using the bipolar electrosurgical instrument comprises the following steps. A surgeon grasps the ring handles 20 and 21 on the instrument 10 to manipulate the jaws 16 and 17. A vessel or vascular tissue is compressed between the opposable seal surfaces 18 and 19. The opposable seal surfaces 18 and 19 preferably come together in aligned opposition due to the alignment action of the open-lockbox 13, or in certain embodiments due to the alignment action of the shoulder pin 34. The surgeon further deflects the shank portions 14 and 15 to engage the ratchet teeth 26 and 27. The engagement of the ratchet teeth 26 and 27 hold the shank portions 14 and 15 in their deflected positions to provide a constant spring force that is transmitted as a closure force to the jaws 16 and 17. The electrosurgical generator 100 is connected to the instrument 10 through connectors 22 and 23 on the ring handles 20 and 21. An electrical switch is used to close a circuit between the generator and the instrument 10. The switch may be a footswitch, handswitch or automated based upon satisfaction of a series of electrical or electro-mechanical parameters. The electrosurgical current flows through an electrically conductive path on each of the inner member 11 and the outer clamping member 12 between their respective electrical connector, 22 or 23, and their respective seal surface, 18 or 19. An electrically insulative coating 36 substantially covers the inner member 11 and the outer clamping member 12, including the outer periphery of the jaws 16 and 17.

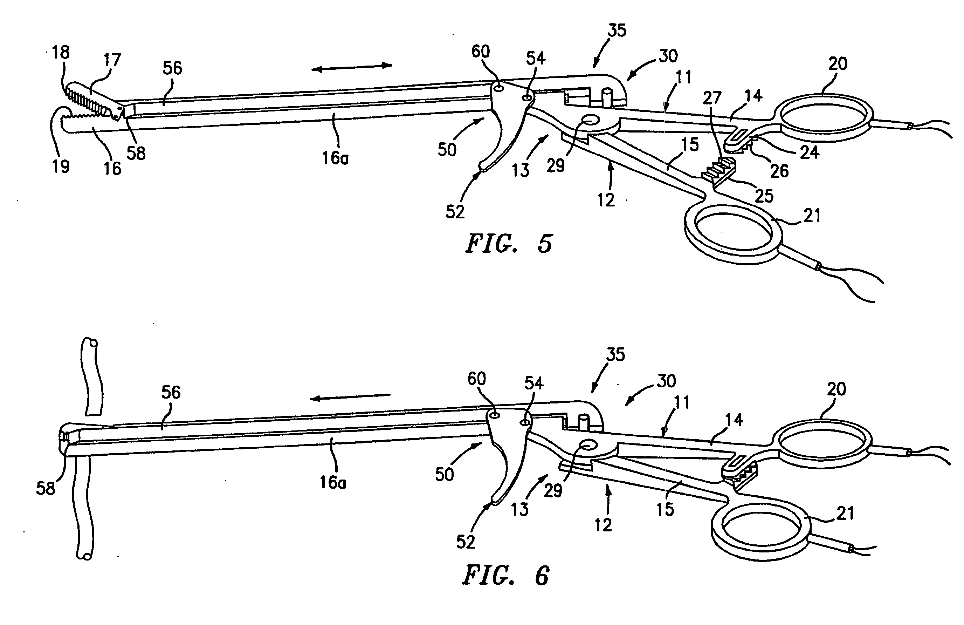

With reference to FIG. 5, there is shown a perspective view of the electrosurgical instrument for vessel fusion in accordance with another preferred embodiment where like reference numerals indicate components identical or similar to the components of the above-described embodiments. This embodiment of the electrosurgical instrument shown by FIG. 5 includes a cutting mechanism 50 having a moveable cutting actuator 52. The cutting actuator 52 is connected to the inner member 11 via a first pin 54 and to a rod 56 having a distal cutting edge 58 via a second pin 60.

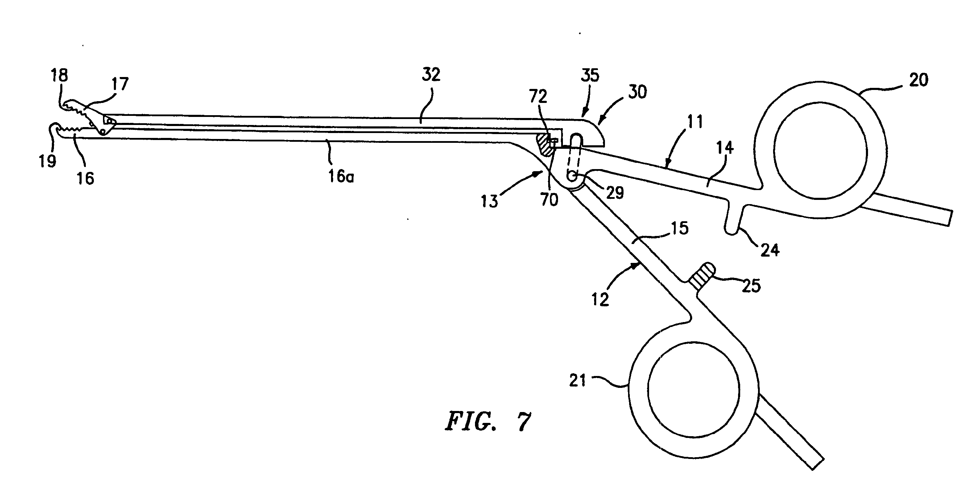

The cutting actuator 52 pivots counter-clockwise with respect to the first pin 54 to push rod 56 distally between sealing surfaces 18 and 19 to cut tissue clamped there between as shown by FIG. 6. The cutting actuator 52 pivots clockwise to pull rod 56 proximally after a cutting procedure. The rod 56 and the distal cutting edge 58 are preferably formed from a unitary piece of metal which is coated with an insulative material to prevent causing a short circuit between the inner member 11 and the outer clamping member 12 and the opposable sealing surfaces 18 and 19.

The rod 56 may be hot-wired by connecting it to the electrosurgical generator 100, or another RF source, for heating the distal cutting edge 58 for cutting tissue by only applying heat thereto (resistive heating) via the cutting edge 58, or for cutting tissue by both applying heat thereto and mechanically cutting the tissue by the cutting edge 58.

The distal cutting edge 58 may be substantially dull having serrated cutting edges to enhance cutting. Further, the cutting-mechanism 50 may include a spring-like actuator (not shown) which rapidly advances the cutting edge 58 through the tissue with a predetermined force which is enough to cut tissue along a seal plane or between two seals.

The electrosurgical instrument of the present disclosure can also have a cutting mechanism having an arcuate cutter recessed in a channel within one of the jaws 16 and 17. The arcuate cutter is able to cut tissue by rotating. It is provided that in order for the cutter to cut tissue along a seal, the surgeon must seal the tissue to form the seal, then regrasp the tissue at a different location so that the cutter cuts along the seal. The cutter can also be longitudinally shaped and recessed within a central portion of one of the jaws 16 and 17. The central portion aligns with the seal. The surgeon can then cut the tissue along the seal by extending the cutter without having to regrasp the tissue.

FIG. 7 illustrates a side view of the electrosurgical instrument for vessel fusion in accordance with another preferred embodiment utilizing different-sized plugs 70 at the proximal end of the electrosurgical instrument for controlling the separation distance between the jaws 16 and 17 at the distal end. The surgeon can select one of the different-sized plugs 70 for fitting within a plug hole 72 for controlling the separation distance between the jaws 16 and 17 depending on the tissue thickness and type. Each plug preferably includes writing on the outer surface indicating the corresponding minimum separation distance if used to control the separation distance.

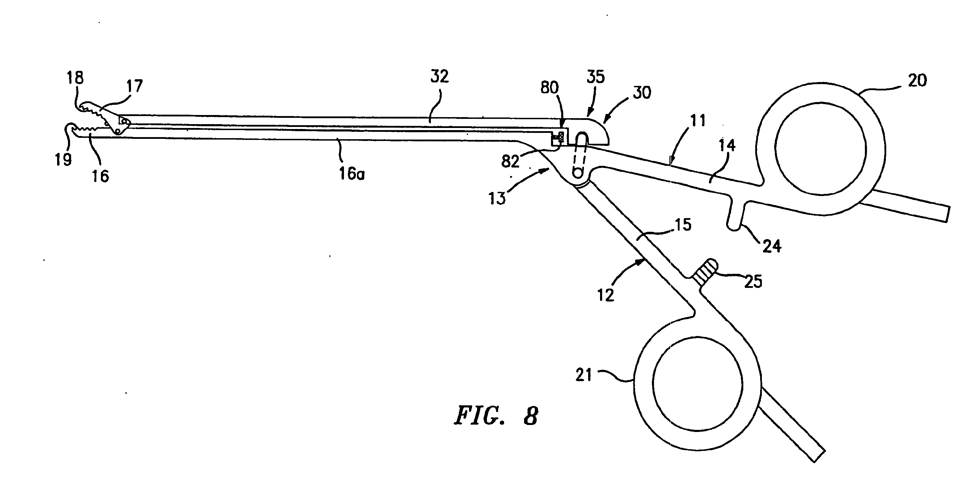

FIG. 8 is a side view of the electrosurgical instrument for vessel fusion in accordance with another preferred embodiment utilizing a screw-like mechanism 80 at the proximal end of the electrosurgical instrument for controlling the separation distance between the jaws 16 and 17 at the distal end. The screw-like mechanism 80 includes a screw 82 capable of being screwed and unscrewed to the inner member 11 for controlling the separation distance between the jaws 16 and 17.

It is appreciated that one skilled in the art can design the electrosurgical instrument of the present disclosure to have a pull-rod mechanism, as opposed to a push-rod mechanism. Such an instrument would be similar in design as the instrument of the present disclosure, but have a stationary outer member and a movable inner member having the pull-rod mechanism. As the movable inner member moves with respect to the stationary outer member, a pull-rod of the pull-rod mechanism would pull one of the sealing jaws to move towards the opposing sealing jaw to seal or fuse vessels, tissue, and the like. It is also appreciated that one skilled in the art can design the electrosurgical instrument of the present disclosure to make the inner member 11 movable and provide the inner member 11 with the open lockbox 13.

From the foregoing and with reference to the various figure drawings, those skilled in the art will appreciate that certain modifications can also be made to the present disclosure without departing from the scope of the present disclosure. For example, it may be preferable to add other features to the instrument 10, e.g., an articulating assembly to axially displace the jaw members 16 and 17 relative to the inner member 11 and outer clamping member 12. The tissue contacting surfaces may include radiused edges to facilitate sealing and reduce possible collateral damage to tissue during sealing. The instrument may include a light source or endoscope which is selectively attachable to the inner member 11 or outer clamping member 12 or other part of the instrument (e.g., proximal the jaw members 16 and 17) or which is integrally associated with one or more parts of the instrument 10. As can be appreciated, the light source may be self contained or connectable to an optical source remotely disposed relative to the operative site.

While several embodiments of the disclosure have been shown in the drawings and described herein, it is not intended that the disclosure be limited thereto, as it is intended that the disclosure be as broad in scope as the art will allow and that the specification be read likewise. Therefore, the above description should not be construed as limiting, but merely as exemplifications of preferred embodiments. Those skilled in the art will envision other modifications within the scope of the claims appended hereto.

## Claims

1. An electrosurgical instrument (10) for sealing tissue, comprising:
an inner member (11) and an outer clamping member (12) at least one of which includes a ring handle (20, 21) near a proximal end thereof, at least one of said inner member (11) and said outer clamping member (12) being movable from a first position wherein two seal surfaces (18, 19) are spaced relative to one another to a second position wherein said seal surfaces (18, 19) are disposed in opposing relation relative to one another for grasping tissue therebetween, wherein said seal surfaces (19, 18) are provided proximate the respective distal ends of said inner member (11) and said outer clamping member (12);
and means (22, 23, 100) for energizing said seal surfaces (18, 19) such that electrosurgical energy can be communicated through tissue held therebetween when said seal surfaces are dispose din said second position to form a tissue seal;
**characterized by**:
a rod (32) operably connected at a proximal end to said outer clamping member (12) and operably connected at a distal end to said seal surface (18) of said outer clamping member (12), said rod (32) being translatable to pivot said seal surface (18) of said outer clamping member (12) relative to said seal surface (19) of said inner member (11),
a stop (36) located near the proximal end of said inner member (11) for limiting distal movement of said push rod (32) and to maintain a gap between said opposing seal surfaces (18, 19) when said seal surfaces (18, 19) are disposed in said second position; and
each of said inner member (11) and said outer clamping member (12) includes a shank portion (14, 15) and ratchets (26, 27), said shank portion (14, 15) being defectable for providing a spring load during tissue clamping and being lockable in a deflected position by means of the ratchets (26, 27) to hold a desired closure force.

2. An electrosurgical instrument (10) for sealing tissue according to claim 1, wherein said ratchets (26, 27) cooperate to hold a closure force at discrete positions within the range of about 3 Kg/cm² to about 16 Kg/cm².

3. An electrosurgical instrument (10) for sealing tissue according to any preceding claim, wherein the rod (32) operatively connects to a pivoting arm (31) which, in turn, operatively connects to the outer clamping member(12) such that movement of the outer clamping member (12) pivots said pivoting arm (31) to move said rod (32).

4. An electrosurgical instrument (10) for sealing tissue according to any preceding claim, wherein said stop (36), maintains a separation distance between said sealing surfaces (18, 19) in the range of about 0.03 millimeters to about 0.15 millimeters.

5. An electrosurgical instrument (10) for sealing tissue according to any preceding claim, wherein said stop (36) is selectively positionable to vary the separation distance between said sealing surfaces (18, 19).

6. An electrosurgical instrument (10) for sealing tissue according to any preceding claim, wherein said stop (36) is selectively replaceable to vary the separation distance between said sealing surfaces (18, 19).

7. An electrosurgical instrument (10) for sealing tissue according to any preceding claim, further comprising a cutting mechanism (50) which is selectively actuateable to sever tissue after formation of the tissue seal.

8. An electrosurgical instrument (10) for sealing tissue, according to any preceding claim, wherein at least one of said inner member (11) and said outer clamping member (12) is pivotable within an open lockbox (13) to move from said first position wherein said seal surfaces (18, 19) are spaced relative to one another to said second position wherein said seal surfaces (18, 19) are disposed in opposing relation relative to one another for grasping tissue therebetween, said open lockbox (13) including a bottom surface disposed on one of said inner member (11) and said outer clamping member (12) which matingly receives a corresponding surface disposed on the other of said inner member (11) and said outer clamping member (17).

9. An electrosurgical instrument (10) for sealing tissue according to claim 8, further comprising a pivot (29) disposed through said bottom surface of said open lockbox (13) for joining the inner member (11) and the outer clamping member(12) for pivotable rotation thereabout.

## Patentansprüche

1. Elektrochirurgisches Instrument (10) zum Verschließen von Gewebe, umfassend:
ein inneres Element (11) und ein äußeres Klammerelement (12), wobei zumindest eines dieser einen Ringgriff (20, 21) nahe dessen proximalen Endes umfasst, wobei entweder das innere Element (11) oder das äußere Klammerelement (12) von einer ersten Position, in der zwei Verschlussoberflächen (18, 19) relativ zueinander voneinander beabstandet sind, in eine zweite Position, in der die Verschlussoberflächen (18, 19) zum Greifen von Gewebe relativ zueinander in einer gegenüberliegenden Beziehung angeordnet sind, bewegbar sind, wobei die Verschlussoberflächen (18, 19) in der Nähe der jeweiligen distalen Enden des inneren Elementes (11) und des äußeren Klammerelementes (12) vorgesehen sind,
und Mittel (22, 23, 100) zur Versorgung der Verschlussoberflächen (18, 19) mit Energie derart, dass elektrochirurgische Energie durch Gewebe, das zwischen jenen gehalten wird wenn die Verschlussoberflächen in der zweiten Position angeordnet sind, hindurch treten kann, um einen Gewebeverschluss auszuformen, **gekennzeichnet durch**: ein Gestänge (32), das betriebsmäßig an einem proximalen Ende mit dem äußeren Klammerelement (12) verbunden ist und
betriebsmäßig an einem distalen Ende mit der Verschlussoberfläche (18) des äußeren Klammerelements (12) verbunden ist, wobei das Gestänge (32) einer Translation derart unterzogen werden kann, dass die Verschlussoberfläche (18) des äußeren Klammerelements (12) relativ zu der Verschlussoberfläche (19) des inneren Elements (11) verkippt, einen Stopper (36), welcher nahe dem proximalen Ende des inneren Elements (11) zur Beschränkung der distalen Bewegung des Gestänges (32) angeordnet ist, und um einen Spalt zwischen den gegenüberliegenden Verschlussoberflächen (18, 19) aufrecht zu erhalten, wenn die Verschlussoberflächen (18, 19) in der zweiten Position angeordnet sind, und wobei sowohl das innere Element (11) als auch das äußere Klammerelement (12) einen Schlüsselabschnitt (14, 15) und Ratschen (26, 27) umfassen, wobei der Schlüsselabschnitt (14, 15) zum Bereitstellen einer Federbelastung während des Gewebeklammerns ablenkbar ist und in einer abgelenkten Position mittels der Ratschen (26, 27) verriegelbar ist, um eine gewünschte Schließkraft aufrecht zu erhalten.

2. Elektrochirurgisches Instrument (10) zum Verschließen von Gewebe gemäß Anspruch 1, wobei die Ratschen (26, 27) miteinander kooperieren, um eine Verschlusskraft in diskreten Positionen innerhalb des Bereiches von ungefähr 3kg/cm² bis ungefähr 16 kg/cm² aufrecht zu erhalten.

3. Elektrochirurgisches Instrument (10) zum Verschließen von Gewebe gemäß einem der vorstehenden Ansprüche, wobei das Gestänge (32) operativ mit einem Verschwenkarm (31) verbunden ist, welcher seinerseits, operativ mit dem äußeren Klammerelement (12) verbunden ist, so dass die Bewegung des äußeren Klammerelements (12) den Verschwenkarm (31) verschwenkt, um das Gestänge (32) zu bewegen.

4. Elektrochirurgisches Instrument (10) zum Verschließen von Gewebe gemäß einem der vorstehenden Ansprüche, wobei der Anschlag (36) einen Teilungsabstand zwischen den Verschlussoberflächen (18, 19) in dem Bereich von ungefähr 0,03mm bis ungefähr 0,15mm aufrechterhält.

5. Elektrochirurgisches Instrument (10) zum Verschließen von Gewebe gemäß einem der vorstehenden Ansprüche, wobei der Stopper (36) so auswählbar positionierbar ist, dass er den Teilungsabstand zwischen den Verschlussoberflächen (18, 19) variiert.

6. Elektrochirurgisches Instrument (10) zum Verschließen von Gewebe gemäß einem der vorstehenden Ansprüche, wobei der Stopper (36) selektiv ersetzbar ist, um den Teilungsabstand zwischen den Verschlussoberflächen (18, 19) zu variieren.

7. Elektrochirurgisches Instrument (10) zum Verschließen von Gewebe gemäß einem der vorstehenden Ansprüche, weiterhin umfassend einen Schneidemechanismus (50) der auswählbar betätigbar ist, um Gewebe nach dem Ausbilden der Gewebedichtung zu schneiden.

8. Elektrochirurgisches Instrument (10) zum Verschließen von Gewebe gemäß einem der vorstehenden Ansprüche, wobei zumindest entweder das innere Element (11) oder das äußere Klammerelement (12) innerhalb einer offenen Verriegelungsbox (13) so verschwenkbar ist, dass sie sich von der ersten Position, in der die Verschlussoberfläche (18, 19) relativ zueinander voneinander beabstandet sind, in die zweite Position, in der die Verschlussoberflächen (18, 19) in einer gegenüberliegenden Beziehung relativ zueinander verschwenkbar sind zum Greifen von dazwischen angeordnetem Gewebe, wobei die offene Verriegelungsbox (13) eine Bodenoberfläche, welche entweder an dem inneren Element (11) oder dem äußeren Klammerelement (12) angeordnet ist, umfasst, welche passend eine korrespondierende Oberfläche, die an dem jeweils anderen des inneren Elements (11) und des äußeren Klammerelements (17) angeordnet ist, aufnimmt.

9. Elektrochirurgisches Instrument (10) zum Verschließen von Gewebe gemäß Anspruch 8, weiterhin umfassend einen Verschwenkstift (29) welcher durch die Bodenoberfläche der offenen Verriegelungsbox (13) hindurch zum Verbinden des inneren Elements (11) und des äußeren Klammerelements (12) für eine verschwenkbare Rotation um diesen herum angeordnet ist.

## Revendications

1. Instrument électrochirurgical (10) pour souder du tissu, comprenant :
un élément intérieur (11) et un élément de serrage extérieur (12), dont au moins un comprend une poignée annulaire (20, 21) près de son extrémité proximale, au moins l'un dudit élément intérieur (11) et dudit élément de serrage extérieur (12) étant déplaçable d'une première position dans laquelle deux surfaces de soudure (18, 19), sont espacées l'une de l'autre à une seconde position où lesdites surfaces de soudure (18, 19) sont disposées en une relation opposée l'une à l'autre pour saisir le tissu entre elles, où lesdites surfaces de soudure (19, 18) sont réalisées à proximité des extrémités distales respectives dudit élément intérieur (11) et dudit élément de serrage extérieur (12) ;
et des moyens (22, 23, 100) pour exciter lesdites surfaces de soudure (18, 19) de sorte que l'énergie électrochirurgicale peut être communiquée à travers le tissu tenu entre elles lorsque lesdites surfaces de soudure sont mises dans ladite seconde position pour former une soudure de tissu ;
**caractérisé par** :
une tige (32) reliée fonctionnellement à une extrémité proximale audit élément de serrage extérieur (12) et reliée fonctionnellement à une extrémité distale à ladite surface de soudure (18) dudit élément de serrage extérieur (12), ladite tige (32) pouvant effectuer à un mouvement de translation pour faire pivoter ladite surface de soudure (18) dudit élément de serrage extérieur (12) relativement à ladite surface de soudure (19) dudit élément intérieur (11),
une butée d'arrêt (36) située près de l'extrémité proximale dudit élément intérieur (11) pour limiter un mouvement distal de ladite tige de poussée (32) et pour maintenir un espace entre lesdites surfaces de soudure opposées (18, 19) lorsque lesdites surfaces de soudure (18, 19) sont disposées dans ladite seconde position ; et
chacun dudit élément intérieur (11) et dudit élément de serrage extérieur (12) comprend une portion de fût (14, 15) et des cliquets (26, 27), ladite portion de fût (14, 15) pouvant être défléchie pour réaliser une charge de ressort pendant le serrage du tissu et pouvant être verrouillée dans une position défléchie au moyen des cliquets (26, 27) pour maintenir une force de fermeture souhaitée.

2. Instrument électrochirurgical (10) pour souder du tissu selon la revendication 1, où lesdits cliquets (26, 27) coopèrent pour maintenir une force de fermeture à des positions discrètes dans la plage d'environ 3 Kg/cm² à environ 16 Kg/cm².

3. Instrument électrochirurgical (10) pour souder du tissu selon l'une des revendications précédentes, où la tige (32) est relié fonctionnellement à un bras de pivotement (31) qui, à son tour, est relié fonctionnellement à l'élément de serrage extérieur (12) de telle sorte que le mouvement de l'élément de serrage extérieur (12) fait pivoter ledit bras de pivotement (31) pour déplacer ladite tige (32).

4. Instrument électrochirurgical (10) pour souder du tissu selon l'une des revendications précédentes, où ladite butée d'arrêt (36) maintient une distance de séparation entre lesdites surfaces de soudure (18, 19) dans la plage d'environ 0,03 millimètre à environ 0,15 millimètre.

5. Instrument électrochirurgical (10) pour souder du tissu selon l'une des revendications précédentes, où ladite butée (36) peut être positionnée sélectivement pour faire varier la distance de séparation entre lesdites surfaces de soudure (18, 19).

6. Instrument électrochirurgical (10) pour souder du tissu selon l'une des revendications précédentes, où ladite butée (36) peut être remplacée sélectivement pour faire varier la distance de séparation entre lesdites surfaces de soudure (18, 19).

7. Instrument électrochirurgical (10) pour souder du tissu selon l'une des revendications précédentes, comprenant en outre un mécanisme de coupe (50) qui est actionnable sélectivement pour couper le tissu après la formation de la soudure de tissu.

8. Instrument électrochirurgical (10) pour souder du tissu selon l'une des revendications précédentes, où au moins l'un dudit élément intérieur (11) et dudit élément de serrage extérieur (12) peut pivoter dans une boîte de verrouillage ouverte (13) pour se déplacer de ladite première position dans laquelle lesdites surfaces de soudure (18, 19) sont espacées l'une de l'autre à ladite seconde position où lesdites surfaces de soudure (18, 19) sont disposées en une relation opposée l'une à l'autre pour saisir le tissu entre elles, ladite boîte de verrouillage ouverte (13) comprenant une surface inférieure disposée sur l'un dudit élément intérieur (11) et dudit élément de serrage extérieur (12) qui reçoit d'une manière correspondante une surface conjuguée disposée sur l'autre dudit élément intérieur et dudit élément de serrage extérieur (17).

9. Instrument électrochirurgical (10) pour souder du tissu selon la revendication 8, comprenant en outre un pivot (29) disposé à travers ladite surface inférieure de ladite boîte de verrouillage ouverte (13) pour relier l'élément intérieur (11) et l'élément de serrage extérieur (12) en vue d'une rotation pivotante autour de celui-ci.
